# EUROPÄISCHE PATENTANMELDUNG

(11) **EP 1 312 316 A2**
(43) Veröffentlichungstag der Anmeldung: **21.05.2003**
(21) Anmeldenummer: 02024670.8
(22) Anmeldetag: 05.11.2002
(51) Int. Cl.: A61B 17/64

(54) **Fixationselement zur Herstellung einer Fixationsverbindung auseinandergebrochener Knochenteile**

(30) Priorität: 14.11.2001 DE 20118441 U
(71) Anmelder: Tantum AG, 24534 Neumünster (DE)
(72) Erfinder: Arpe, Michael, 24105 Kiel (DE); Mamero, Thomas, 24105 Kiel (DE); Jensen, Ham-Iven, 24214 Noer (DE)
(74) Vertreter: Biehl, Christian, Dipl.-Phys.

(57) **Zusammenfassung**

Fixationselemente zur Herstellung einer Fixationsverbindung wenigstens zweier auseinandergebrochener Knochenteile durch Verbindung von Knochennägeln (18) mit Längselementen, die in den Fixationselemten gelagert sind, in einstellbaren festen Winkeln, mit wenigstens einem ersten Fixationselement (10) mit wenigstens einem Aufnahmeschlitz (16) für wenigstens einen Knochennagel (18) und mit wenigstens einem rotationssymmetrischen Klemmverbindungssitz (12), wenigstens einem weiteren, zweiten Fixationselement (22) zur Aufnahme und Halterung von Fixationsstangen (42) über die Knochenbruchstelle zu einem weiteren zweiten Fixationselement (22), das wiederum über ein erstes Fixationselement (10) an einem oder mehr Knochennägeln (18) befestigt ist, und das ein Paßteil (28) mit entsprechend dem Klemmverbindungssitz (12) im ersten Fixationselement (10) ausgebildeten Abmessungen aufweist, wobei der Klemmverbindungssitz (12) mit Formschlußmitteln zur Arretierung des Paßteils (28) in einer bestimmten Winkelstellung bei hergestellter Klemmverbindung versehen ist.

## Beschreibung

Die Erfindung betrifft Fixationselemente nach dem Oberbegriff des Hauptanspruches.

Fixationselemente werden beim Herstellen von Fixationsverbindungen nach Knochenbrüchen an Knochennägeln angesetzt, die in den jeweiligen Knochenteilen eingebracht sind, und dienen zur Halterung von Fixationsstangen, die über die Bruchstelle(n) hinweg mit weiteren Fixationselementen in Knochennägeln in Verbindung stehen, um ein gerades Zusammenwachsen der Knochenteile in richtiger Stellung zueinander zu ermöglichen.

Abhängig von der Ausrichtung der Knochennägel und der zu überbrückenden Strecke müssen unterschiedliche Winkel fest eingestellt werden, die sich dann lange Zeit - bis zur Ausheilung des Knochenbruches - nicht verstellen dürfen. Bisher wird dies über ein Metallgestänge mit Klemmschrauben erreicht, die in verbleichsweise schweren Verbindungselementen befestigt, kraftschlüssig auf Metallflächen pressen. Diese Metallstäbe und Verbindungelemente werden wegen ihres Materialwertes wiederverwendet, wobei allerdings die Sterilisation kostenaufwendig ist.

Der Erfindung liegt die Aufgabe zugrunde, ein vom Gewicht für den Patienten leichteres, einfacher für den Arzt bedienbares System zu schaffen, daß dennoch sicher die Fixation ermöglicht.

Erfindungsgemäß wird dies durch ein Einwegsystem, vorteilhafterweise mit aus Kunststoff gebildeten Fixationselementen gelöst, daß die Merkmale des Hauptanspruches besitzt. Die Unteransprüche geben vorteilhafte Ausführungsformen der Erfindung und ein Verfahren zum Herstellen einer Fixationsverbindung mit diesen Fixationselementen wieder.

Durch den leichteren Werkstoff erhöht sich der Tragekomfort und durch die Einmalverwendung fallen alle Probleme mit der Sterilisation weg.

Vorteilhaft ist insbesondere, daß durch den Verzicht auf Schraubklemmen ein leichteres Material der Fixationselemente gewählt werden kann, ohne die Tragkraft der Fixationsverbindung zu verringern. Es ist nicht mehr notwendig, nur kostenträchtig sterilisierbare Gewindegänge aus einem metallischen Werkstoff herzustellen. Das geringere Gesamtgewicht verbunden mit dem Verzicht auf viele Ecken und Vorsprünge (durch Klemmschrauben) erleichtert das Tragen der Fixationsverbindung für den Patienten erheblich.

Durch die Schaffung von Klemmverbindungen, die sich selbst in einer Winkelposition fixieren und die - was die Bedienung erleichtert - beim Herstellen der Klemmverbindung noch bis zum letzten Moment vor dem Klemmen einstellbar bleiben, ist ein schnelles, genau ausgerichtetes Zusammensetzen der Fixationsverbindung erleichtert.

Für die Dauer des Tragens werden die eingestellten Winkel durch auf die Fixationselemente mit einer Prägezange aufgeprägte Klemmringe gesichert, so daß sichergestellt ist, daß die vom Arzt eingestellte Fixationsverbindung sich weder nachträglich lockern kann, noch unbefugt lockern läßt.

Weitere Vorteile und Merkmale der Erfindung ergeben sich aus nachfolgender Beschreibung eines bevorzugten Ausführungsbeispiels anhand der beigefügten Zeichnung. Dabei zeigt:
- Fig. 1: zwei schematisch in einen Arm eingebrachte Knochennägel und ein erstes Fixationselement, daß zur Aufnahme der freien Enden der Knochennägel mit zwei Längsschlitzen an seinen Enden versehen ist,
- Fig. 2: ein in ein erstes Fixationselement der Fig. 1 zu steckendes zweites Fixationselement zur Einlagerung in den Metallbereich des ersten Fixationselementes,
- Fig. 3: ein entsprechend wie in der Fig. 1 auf den Knochennägeln befestigtes erstes Fixationselement und ein zweites Fixationselement, durch das die zu haltende Fixationsstange verläuft,
- Fig. 4: eine schematische Schnittzeichnung die die beiden Fixationselemente beim Zusammenstecken zeigt,
- Fig.5: eine schematische Schnittzeichnung die die Freiheitsgrade der Verdrehung einer Fixationsstange mit aufgesetzter Kugelhülse nach dem Zusammenstecken, noch vor dem Verklemmen mit einer Klemmhülse zeigt,
- Fig. 6: eine Draufsicht auf einen beispielhaften Klemmhülsenring, und
- Fig. 7: eine seitliche Ansicht auf einen Klemmhülsenring.

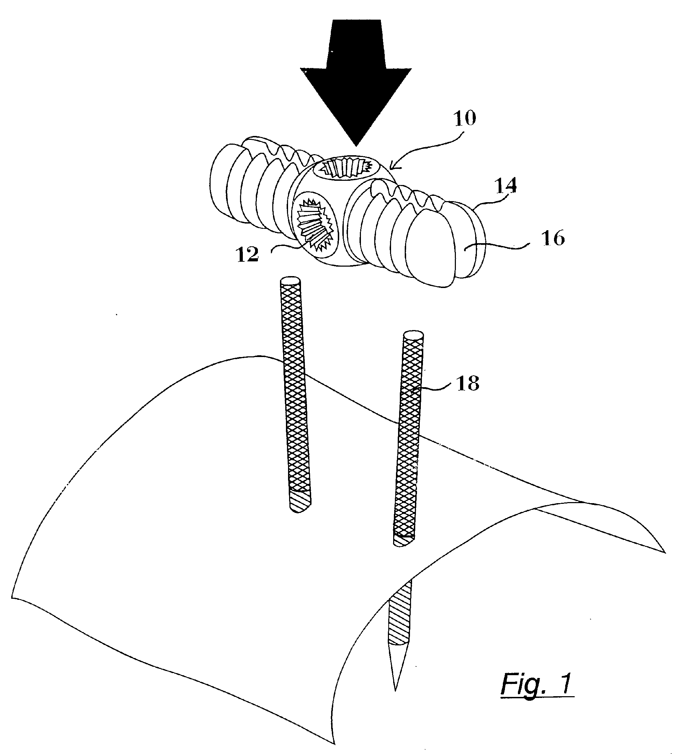

Das in der Fig. 1 dargestellte erste Fixationselement 10 weist neben einem Mittelbereich, in den konische Aufnahmen 12 eingeformt sind, zwei im wesentlichen zylindrische Außenbereiche auf, in die je ein Längsschlitz bis zur Stirnfläche 16 eingebracht ist, der zur Aufnahme der Knochennägel 18 dient. Insgesamt ergibt sich eine knebelartige Form mit zwei Ansätzen, bei der alle Kanten angerundet sind.

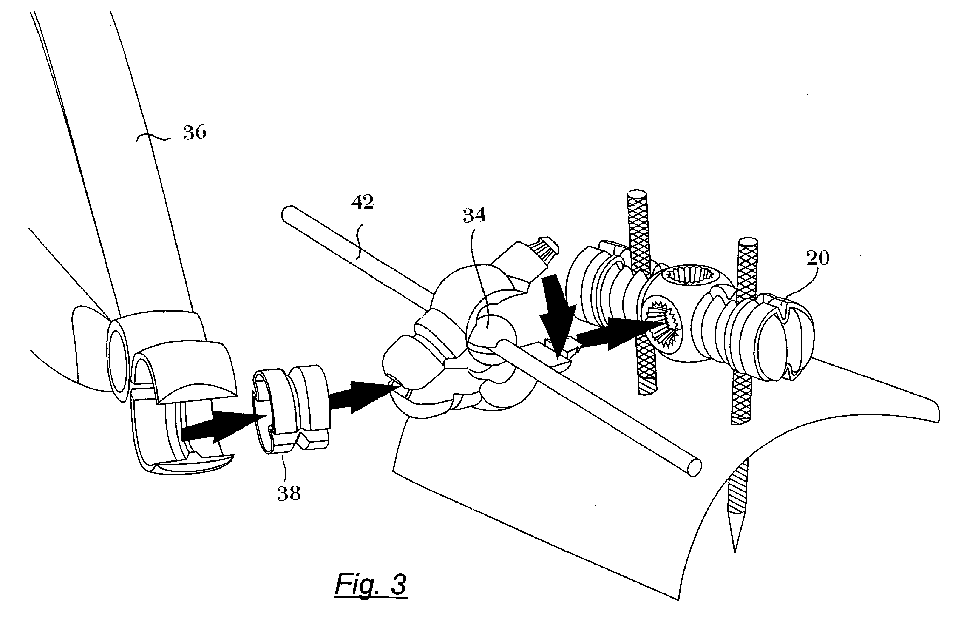

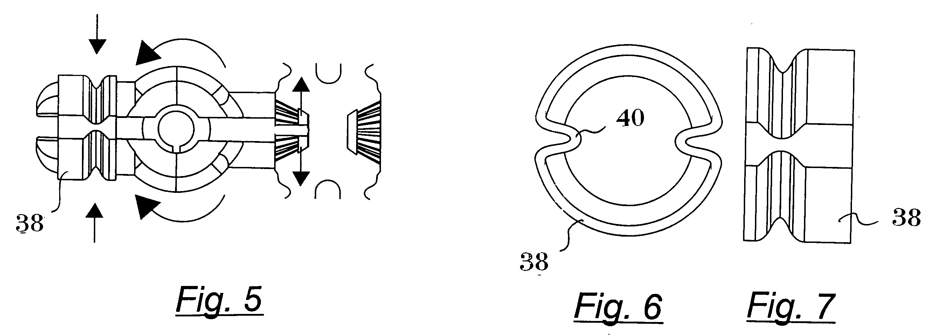

Nicht dargestellt sind in der Fig. 1 die erst in der Fig. 3 erkennbaren, (und in Fig. 6 und 7 im Detail am Beispiel der ähnlichen Klemmhülsenringe dargestellten) auf die rippenartige Außenprofilierung der zylindrischen Abschnitte aufpassenden Prägehülsen 20, die zur Fixierung des ersten Fixationselementes an den Knochennägeln dienen. Ein einfacher zylindrischer Klemmring anstelle der Prägehülsen 20, der leicht elliptisch verformt wird, erfüllt die Aufgabe der Sicherung aber ebenso. Diese Prägehülsen oder Klemmringe werden mit einer Zange vor dem Ansatz weiterer Elemente zunächst unter Einklemmung der Knochennägel bei Verpressung der Längsschlitzinnenseiten an die (profilierten) Knochennägel angebracht.

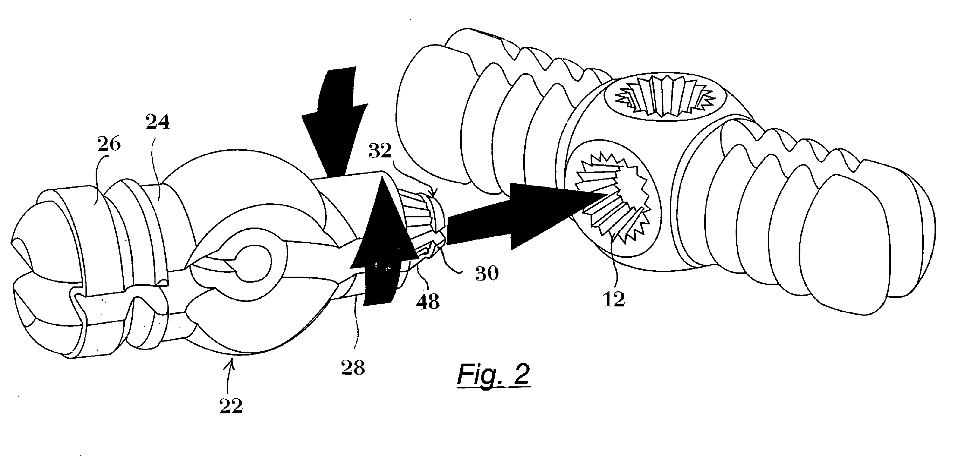

In der Fig. 2 ist neben dem ersten Fixationselement ein zweites Fixationselement 22 dargestellt, das neben einem Mittelbereich zur Umfassung einer Fixationsstange einen zylindrischen Abschnitt 24 aufweist, auf den wieder ein Prägering 26 zur Befestigung aufgebracht werden kann und gegenüberliegend diesem einen weiteren im wesentlichen zylindrischen Abschnitt 28 besitzt, an dessen Ende ein konischer, sich im wesentlichen verjüngender Klemmabschnitt 32 mit einem am Ende wieder aufgeweiteten Kopf 30 vorgesehen ist.

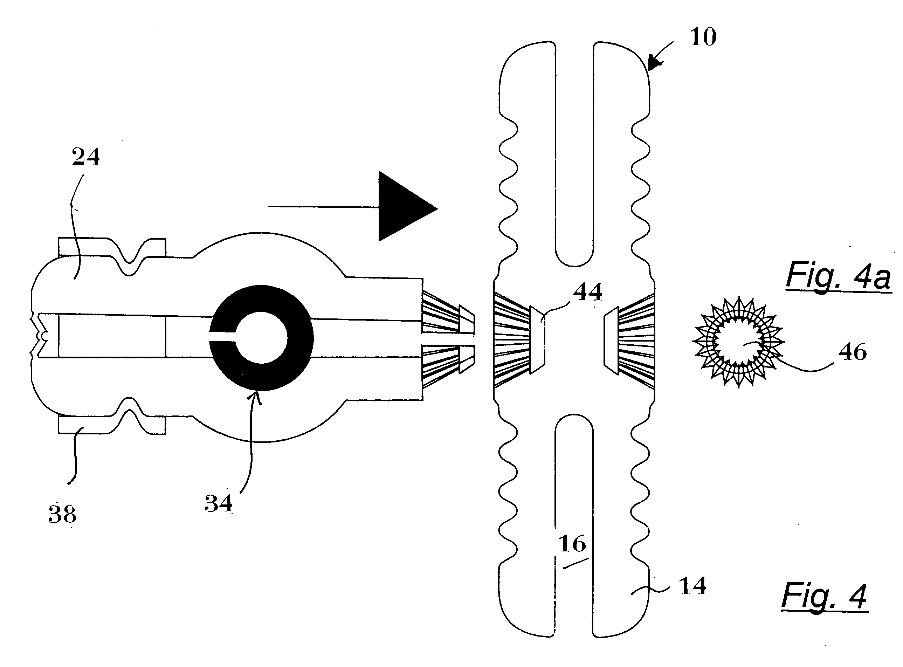

Dieser Klemmabschnitt ist entsprechend der konischen Aufnahme 12 des ersten Fixationselementes konisch mit einer entsprechenden Profilierung (in der Fig. 4a als Profilierung der Aufnahme 12 dargestellt) in Form bevorzugt einer Längsverzahnung versehen, um einen Formschluß gegen Verdrehen im eingeklemmten Zustand vorzusehen.

Da vor dem Bewirken der Klemmwirkung des pilzförmigen, zweiteilig sich aufspreizenden Kopfes zunächst eine nicht profilierte Endfläche des Kopfabschnittes 30 auf der Profilierung des ersten Fixationselementes verdrehlich aufliegt, kann vor einem Herstellen der endgültigen Winkellage (durch Hindurchschieben des pilzartig erweiterten Kopfes unter Zusammenbringen seiner beiden Hälften über die zwischen ihnen freigelassene Spaltbreite) bis zum Augenblick des Verklemmens noch frei das zweite Fixationselement gegen das erste Fixationselement gedreht werden.

Es wird vorgeschlagen, daß zweite Fixationselement insgesamt aus zwei Halbschalen zu fertigen, die ggf. noch über Filmscharniere im Abschnitt 24 zur leichteren Handhabung zusammenhängen.

Dadurch kann auch eine längere Fixationsstange von dem zweiten Fixationselement umfaßt werden, wie dies in der Fig. 3 dargestellt ist, wobei zusätzlich noch zur Herstellung beliebiger Winkel der Fixationsstange im zweiten Fixationselement eine auf der Fixationsstange verschiebliche Kugelhülse zum Einsatz kommen kann, die in einer entsprechend kugelförmig geformten Mittelaufnahme des zweiten Fixationselementes gehalten wird. In der Fig. 4 ist die Kugelhülse schwarz dargestellt. Auch sie weist entlang der Stange einen Schlitz auf, um auf die Stange aufpreßbar zu sein.

Im linken Teil der Fig. 3 ist eine Klemmzange 36 für die Klemmringe 38 dargestellt, die die nach Einbringen des zweiten Fixationselementes ins erste Element sich ergebende Belastung auf das Füllscharnier aufrängt und das zweite Fixationselement sicher schließt und gleichzeitig einen Pressdruck auf die Kugelhülse 34 ausübt.

Fig. 5 zeigt die geometrischen Verhältnisse vor einem Verpressen der Klemmringe 38.

Es wird vorgeschlagen, an dem ersten Fixationselement zwei Haltestangen mit zwei zweiten Fixationselementen (auf jeder Seite eines) vorzusehen, um einen sicheren Halt der Knochenteile zueinander durch zwei über die Bruchstelle verlaufende Stangen zu erhalten. Eine dritte Aufnahme an der Oberseite des ersten Fixationselementes kann bei besonders komplizierten Brüchen zur Aufnahme weiterer Fixationselemente zur weiteren Anordnung zusätzlicher Stangen über die zweite Fixationsstange hinaus dienen.

Fig. 6 und 7 zeigen eine der vorgeschlagenen Formen der Klemmringe 38, die neben einer umlaufenden Ringmulde zum festen Sitz in einer Mulde des Abschnittes 24 oder einer der Mulden des Abschnittes 14 des ersten Fixationselementes noch mit zwei nach innen kragenden Quetschbereichen (die sich in die Spaltbreiten und Schlitzabstände der Abschnitte 14 und 24 einlagern) versehen ist. Ein runder Aluminiumring, wie er in alternativen Ausführungen zum Einsatz kommt, ist nicht dargestellt, wird aber ebenso sicheren Sitz garantieren.

Neben den miteinander wirkenden zwei unterschiedlichen Fixationselementen zur Herstellung einer Fixationsverbindung wenigstens zweier auseinandergebrochener Knochenteile durch Verbindung von Knochennägeln 18 mit Längselementen, die in den Fixationselemten gelagert sind, in einstellbaren festen Winkeln, wie sie durch die ersten drei Ansprüche geschützt werden, sind auch die einzelnen Fixationselemente Gegenstand des Schutzes, insbesondere ein erstes Fixationselement 10 mit wenigstens einem Aufnahmeschlitz 16 für wenigstens einen Knochennagel 18 und mit wenigstens einem rotationssymmetrischen Klemmverbindungssitz 12, aber auch das zweite Fixationselement 22 zur Aufnahme und Halterung von Fixationsstangen 42 über die Knochenbruchstelle zu einem weiteren zweiten Fixationselement 22, das nur bevorzugt über ein erstes Fixationselement 10 an einem oder mehr Knochennägeln 18 befestigt ist, und das ein Paßteil 28, 48 mit entsprechend dem Klemmverbindungssitz 12 im ersten Fixationselement 10 ausgebildeten Abmessungen aufweist, wobei der Klemmverbindungssitz 12 mit Formschlußmitteln zur Arretierung des Paßteils 28, 48 in einer bestimmten Winkelstellung bei hergestellter Klemmverbindung versehen ist.

Dabei werden zweite Fixationselemente 22 bevorzugt, die einen zum Teil geschlitzten mit Hinterschneidungen versehenen pilzförmigen Kopf 32 als Klemmteil aufweisen, der durch eine im Klemmsitz 12 der ersten Fixationselemente 10 befindliche Ausnehmung 46 (Fig. 4a) hindurch in einen entsprechend gebildeten Freiraum 44 im ersten Fixationselement 10 einlagerbar ist. Die Formschlußmittel an der Klemmverbindung werden dabei wiederum bevorzugt durch eine konische Hohlkegelfläche mit Zahnkanten längs der Außenseiten des Konus und ein Paßteil 28 hinter dem Kopf 32 bestehen, der einen entsprechenden konischen Abschnitt 48 mit entsprechenden Kerben aufweist.

Das zweites Fixationselement besitzt zur Ermöglichung des freien Verdrehens vor dem arretieren eine angeschrägte Endfläche auf dem pilzförmigen Klemmkopfes 32, die im wesentlichen glatt, d.h. ohne Zahnkanten, ausgebildet ist. Das zweites Fixationselement besteht dabei weiter aus im wesentlichen zwei identischen Halbschalen, die in einem Mittelteil zur Einklemmung einer Kugel mit einem kugeligen Freiraum versehen sind, und an ihrem dem Pilzkopf 32 gegenüberliegenden Ende mit einem Paßsitz für einen Prägering 38 versehen sind.

Bei den ersten Fixationselementen werden in einer bevorzugten Ausführung neben einem Mittelteil mit wenigstens drei Aufnahmen 12 für zweite Fixationselemente 22 zwei im wesentlichen aus zwei aneinander liegenden Halbzylindern 14 gebildete Endabschnitte mit einer Anzahl von nebeneinander liegenden Prägeringaufnahmen zur Fixierung des ersten Elementes an unterschiedlich beabstandeten zwei Knochennägeln 18 vorgesehen.

## Patentansprüche

1. Fixationselemente zur Herstellung einer Fixationsverbindung wenigstens zweier auseinandergebrochener Knochenteile durch Verbindung von Knochennägeln (18) mit Längselementen, die in den Fixationselemten gelagert sind, in einstellbaren festen Winkeln, **gekennzeichnet durch**
ein erstes Fixationselement (10) mit wenigstens einem Aufnahmeschlitz (16) für wenigstens einen Knochennagel (18) und mit wenigstens einem rotationssymmetrischen Klemmverbindungssitz (12),
wenigstens ein weiteres, zweites Fixationselement (22) zur Aufnahme und Halterung von Fixationsstangen (42) über die Knochenbruchstelle zu einem weiteren zweiten Fixationselement (22), das wiederum über ein erstes Fixationselement (10) an einem oder mehr Knochennägeln (18) befestigt ist, und das ein Paßteil (28) mit entsprechend dem Klemmverbindungssitz (12) im ersten Fixationselement (10) ausgebildeten Abmessungen aufweist,
wobei der Klemmverbindungssitz (12) mit Formschlußmitteln zur Arretierung des Paßteils (28) in einer bestimmten Winkelstellung bei hergestellter Klemmverbindung versehen ist.

2. Fixationselemente nach Anspruch 1, **dadurch gekennzeichnet, daß** die zweiten Fixationselemente (22) einen zum Teil geschlitzten mit Hinterschneidungen versehenen pilzförmigen Kopf (32) als Klemmteil aufweisen, der durch eine im Klemmsitz (12) der ersten Fixationselemente (10) befindliche Ausnehmung (46) hindurch in einen entsprechend gebildeten Freiraum (44) im ersten Fixationselement (10) einlagerbar ist.

3. Fixationselemente nach einem der vorangehenden Ansprüche, **dadurch gekennzeichnet, daß** die Formschlußmittel an der Klemmverbindung eine konische Hohlkegelfläche mit Zahnkanten längs der Außenseiten des Konus aufweisen und ein Paßteil (28) hinter dem Kopf einen entsprechenden konischen Abschnitt (48) mit entsprechenden Kerben aufweist.

4. Zweites Fixationselement nach Anspruch 3, **dadurch gekennzeichnet, daß** die Endfläche des pilzförmigen Klemmkopfes (32) glatt, ohne Zahnkanten, ausgebildet ist.

5. Zweites Fixationselement nach einem der vorangehenden Ansprüche, **gekennzeichnet durch** eine aus im wesentlichen zwei identischen Halbschalen gebildete, zweiteilige Ausführung der zweiten Fixationselemente, die in einem Mittelteil zur Einklemmung einer Kugel mit einem kugeligen Freiraum versehen sind, und an ihrem dem Pilzkopf (32) gegenüberliegenden Ende mit einem Paßsitz für einen Prägering (38) versehen sind.

6. Erstes Fixationselement nach einem der vorangehenden Ansprüche, **dadurch gekennzeichnet, daß** neben einem Mittelteil mit wenigstens drei Aufnahmen (12) für zweite Fixationselemente (22) zwei im wesentlichen aus zwei aneinander liegenden Halbzylindern (14) gebildete Endabschnitte mit einer Anzahl von nebeneinander liegenden Prägeringaufnahmen zur Fixierung des ersten Elementes an unterschiedlich beabstandeten zwei Knochennägeln (18) versehen sind.
